# FASCICULE DE BREVET EUROPEEN

(11) **EP 0 502 792 B1**
(45) Date de publication et mention de la délivrance du brevet: **25.11.1998**
(21) Numéro de dépôt: 92400597.8
(22) Date de dépôt: 06.03.1992
(51) Int. Cl.: A61F 5/02

(54) **Corset formant siège pour handicapé physique, notamment pour enfant**
Korsett in Form eines Sitzes für Körperbehinderte, insbesondere für ein Kind
Corset forming a seat for disabled person, in particular for child

(30) Priorité: 07.03.1991 FR 9102739
(43) Date de publication de la demande: 09.09.1992
(73) Titulaire: ETABLISSEMENTS PROTEOR Société anonyme dite:, 21100 Dijon (FR)
(72) Inventeur: Callens, Philippe, F-35590 Clayes (FR)
(74) Mandataire: Jolly, Jean-Pierre

(56) Documents cités:
- EP-A- 0 389 379
- DE-A- 3 522 533
- DE-A- 3 643 540
- FR-A- 2 426 453
- US-A- 4 300 249

## Description

La présente invention concerne un corset formant siège pour handicapés physiques, notamment pour enfants.

Des corsets de ce type sont connus dans la technique depuis environ quinze ans et sont destinés, par exemple, à assister des paraplégiques ou des infirmes moteur cérébraux, en vue de les maintenir en position de correction. Ces corsets sont constitués habituellement d'une demi-coquille en un matériau plastique rigide, qui épouse la forme du siège, des cuisses, du bassin et du tronc du patient, ainsi qu'éventuellement des jambes, des bras et de la tête. Le patient peut reposer simplement dans le siège monobloc ainsi réalisé, ou en être rendu solidaire par des sangles, serrées, lâches or élastiques, par des bretelles ou par des moyens similaires. Ces sangles peuvent être solidaires du corset et être engagées par exemple dans des passants portés par le siège ou des ouvertures ménagées dans celui-ci. Elles peuvent aussi être indépendantes du siège.

Le problème posé par de tels corsets formant siège est que l'enfant ou l'adulte qui y prend appui est maintenu en position fixe, tant au niveau des jambes que des bras, ce qui limite les mouvements des membres et du tronc. Il risque ainsi d'avoir une sensation d'emprisonnement, se traduisant par l'apparition de mouvements spastiques. De plus, il ne peut pas se pencher en arrière ou de côté, ni tendre les bras latéralement pour saisir un objet, ni encore écarter les jambes l'une de l'autre, ce qui accroît les risques de contractures.

Ces inconvénients sont particulièrement sensibles dans le cas d'un enfant handicapé, dont la découverte de l'environnement s'effectue par le regard et qui, une fois qu'il a pris conscience des formes des objets qui l'entourent, tente de les saisir. Son éveil est d'autant plus précoce et d'autant plus important qu'il a été assis tôt, mais s'il est prisonnier d'un corset-siège, son éveil sera plus tardif et plus difficile du fait de la limitation de ses mouvements à un environnement très proche, limité par la coquille du siège.

On connaît par ailleurs par US-A-4 300 249 un siège pour handicapés comportant une ossature sur laquelle prend appui, par l'intermédiaire de ressorts, un siège comprenant diverses parties articulées entre elles.

DE-A-3 643 540 décrit également un siège pour handicapés comportant une coquille rigide intérieure et une coquille rigide extérieure, articulées entre elles par une charnière.

Enfin, EP-A-389 379 décrit une orthèse pour la réduction des scolioses, qui comprend deux parties latérales réunies par une sangle et destinées à enserrer le thorax du patient, une ceinture pelvienne destinée à être rendue solidaire du bassin du patient, et deux tuteurs latéraux déformables élastiquement, qui réunissent les parties latérales et la ceinture pelvienne.

La présente invention vise à proposer un corset formant siège, autorisant au patient une certaine liberté de mouvements.

Un premier but de l'invention est donc de proposer un corset formant siège, dans lequel le patient dispose d'une certaine autonomie de ses mouvements.

Un autre but de l'invention est de proposer un siège de ce type qui soit particulièrement bien adapté aux enfants et qui favorise leur éveil.

Un autre but de l'invention est enfin de proposer un siège de ce type qui puisse être réalisé moyennant un coût réduit par des moyens faciles à mettre en oeuvre.

A cet effet, l'invention a pour objet un corset formant siège tel que décrit dans la revendication 1.

Les parties du corset ainsi rattachées par les organes de liaison déformables élastiquement pourront ainsi être déplacées par le patient les unes relativement aux autres, les organes de liaison déformables élastiquement agissant ainsi comme de véritables articulations munies de moyens de rappel.

Ce ou ces organes pourront être distincts des parties du corset qu'ils réunissent et être fixés sur celles-ci par des moyens connus dans la technique. Ils pourront alors être constitués, par exemple, par des bandes rigides ou tuteurs en matière plastique, éventuellement à mémoire de forme, par des sangles élastiques, par des charnières à ressort, par des éléments en caoutchouc synthétique ou similaires.

Le ou les organes de liaison peuvent également faire partie intégrante du siège-corset et être constitués par une partie de celui-ci dont la forme, les dimensions et/ou l'épaisseur soient telles qu'elles lui permettent de se déformer élastiquement.

Toutes les parties du corset formant siège peuvent être articulées entre elles de cette manière, par exemple, la partie formant siège et celle correspondante à la base du dos du patient, cette dernière et la partie du haut du dos, cette dernière partie et la partie correspondant à la tête, la partie du bas du dos et les parties latérales recevant les bras, la partie du bas du dos et les parties recevant les cuisses, ces dernières et les parties recevant la partie inférieure des jambes etc...

Des formes de réalisation de l'invention vont être décrites ci-après, à titre d'exemples non limitatifs. Dans cette description, on se réfèrent aux dessins annexés, sur lesquels :
La figure 1 représente un corset formant siège de la technique antérieure ;
Les figures 2 et 3 sont des vues en perspective d'un corset formant siège conforme à l'invention, respectivement de face et de l'arrière ;
La figure 4 est une vue analogue à la figure 2 d'une seconde forme de mise en oeuvre de l'invention ;
La figure 5 est une vue analogue à la figure 3 d'une troisième forme de réalisation.

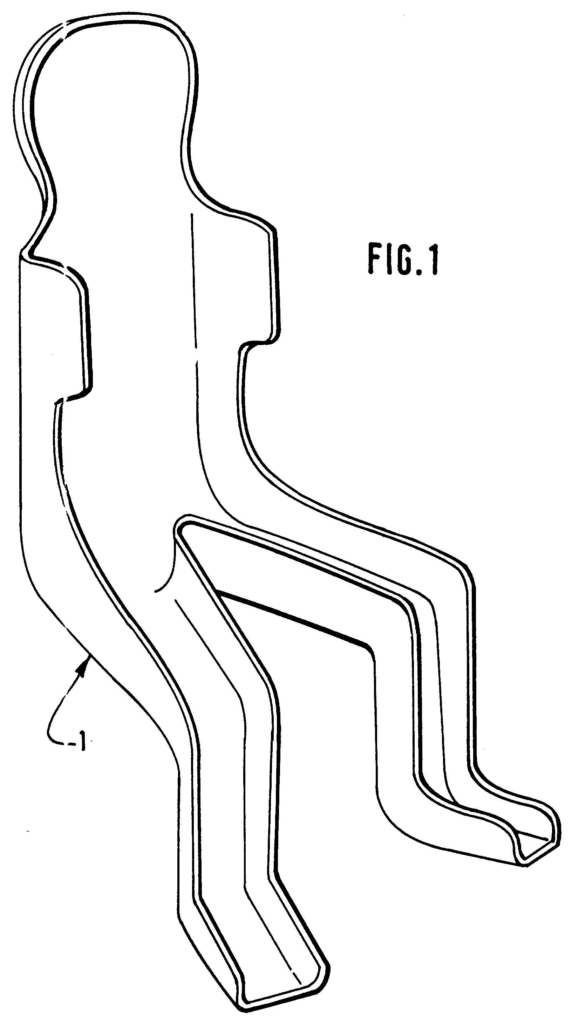

Comme on le voit sur la figure 1, les sièges-corsets 1 de la technique connue sont constitués par une coquille ouverte en matière plastique dans laquelle repose le malade en position assise. Cette coquille peut s'étendre plus ou moins vers le bas ou vers le haut suivant le degré d'autonomie du malade, mais elle comprend généralement une partie formant siège supportant le patient, une partie enserrant le bassin, une partie supportant les cuisses en position écartée et une partie maintenant latéralement les bras, toutes ces parties étant rigidement solidaires les unes des autres et formant un corset monobloc.

Comme il est indiqué ci-dessus, des sangles, des bretelles ou des organes similaires peuvent être prévus pour maintenir le malade en position sur son siège.

Il est clair que, dans un tel corset formant siège, le degré d'autonomie du patient est extrêmement limité, puisqu'il ne peut ni mouvoir les jambes, ni se pencher latéralement pour saisir un objet, ni se pencher en arrière. Ceci est naturellement néfaste à l'état général du patient, qu'il s'agisse, selon le cas, d'un adulte ou d'un enfant, mais, dans ce dernier cas, comme il a déjà été exposé, cette absence de liberté de mouvement est particulièrement regrettable, car elle contrarie l'éveil de l'enfant et sa prise de conscience du monde l'environnant.

Pour remédier à cet inconvénient, l'invention propose de ne plus réaliser le corset formant siège sous forme monolithique, mais en diverses parties, réunies entre elles par des organes de liaison déformables élastiquement. Ces organes de liaison peuvent aussi recevoir un dispositif mécanique ou autre permettant à ceux-ci des angulations différentes autorisant une modification de posture au niveau du dos.

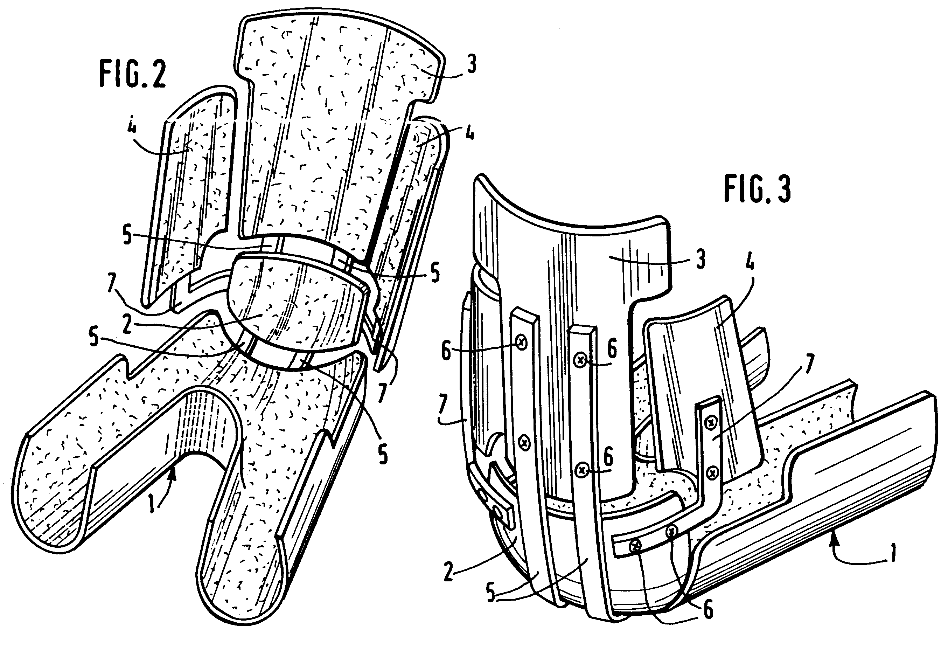

Comme on le voit sur les figures 2 et 3, le corset peut alors comprendre, par exemple, une partie 1 formant siège et supportant les cuisses du patient, une partie 2 servant d'appui au bassin, une partie 3 formant dossier et deux parties 4, servant d'éléments de maintien latéral du patient au niveau des bras, toutes ces parties étant en une matière plastique ou tout autre matériau, rigide ou semi-rigide. Dans cet exemple, les parties 1, 2 et 3 sont réunies par deux bandes 5, dites "tuteurs" dans la technique, en une matière plastique déformable élastiquement, qui sont fixées par des vis ou des rivets 6 à l'arrière de ces parties. Au lieu de tuteurs communs aux parties 1,2 et 3, on pourrait naturellement utiliser des tuteurs distincts réunissant respectivement les parties 1 et 2 et les parties 2 et 3.

De façon analogue, des tuteurs 7, ici en forme d'équerre, également en une matière déformable élastiquement, réunissent les parties 2 et 4, sur les faces arrière desquelles ils sont fixés par des vis ou des rivets 6.

Le patient peut ainsi repousser les parties 2 et 3 s'il désire se pencher en arrière, ou les parties 4 s'il désire se pencher latéralement et/ou déplacer son bras pour saisir, par exemple, un objet. C'est ainsi sous la sollicitation du patient lui-même que les parties constitutives du corset siège sont sollicitées, ce qui constitue un avantage important dans le cas d'un enfant qui découvre son environnement, les parties du corset déplacées revenant ensuite d'elles-même en position, lorsqu'elles ne sont plus sollicitées.

La nature des parties articulées entre elles et le type des organes de liaison dépendent naturellement du handicap physique dont est affecté le patient.

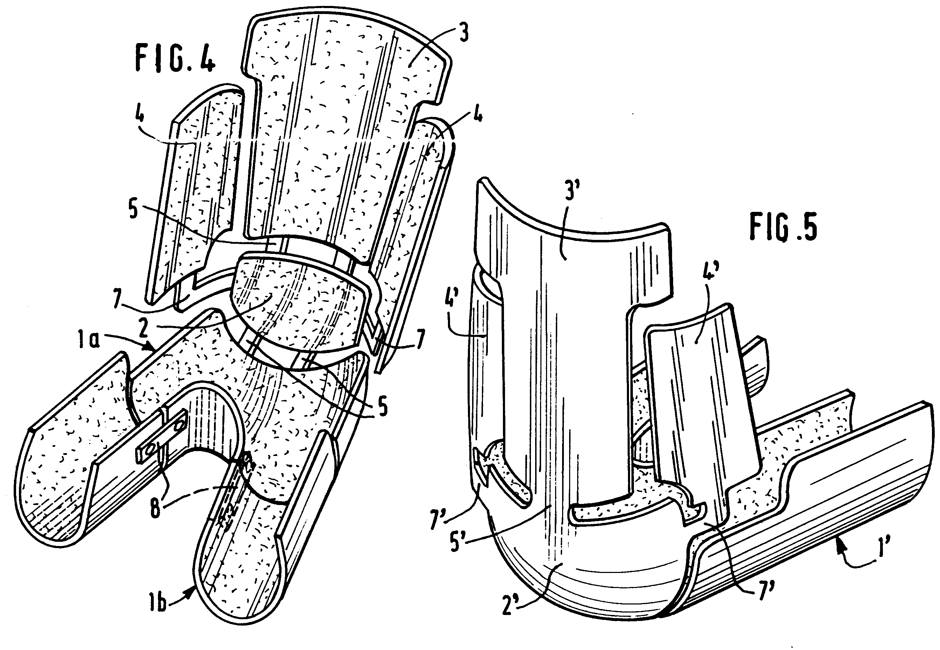

C'est ainsi que, s'il est nécessaire qu'il puisse déplacer ses jambes l'une par rapport à l'autre, la partie 1a formant siège sera distincte de la partie 1b supportant les cuisses du patient et elles seront réunies par des barrettes ou tuteurs 8, comme on le voit sur la figure 4, où les organes déjà décrits en relation avec les figures 2 et 3 sont désignés par les mêmes chiffres de référence.

Comme représenté sur la figure 5, il est également possible de réaliser le corset formant siège sous une forme monobloc. Dans ce cas, le corset est réalisé en une matière semi-rigide à déformation élastique et les différentes parties - partie 1′ supportant le siège du patient, partie 2′ d'appui du bassin, partie 3′ formant dossier et parties 4′ de maintien latéral, dans le cas de la figure 5 - sont réunies entre elles par des attaches telles que 5′ pour les parties 2′ et 3′, et 7′ pour les parties 2′ et 4′, qui font corps avec ces parties et dont la forme et l'épaisseur sont telles qu'elles puissent se déformer élastiquement.

Comme il a été indiqué ci-dessus, on peut naturellement substituer aux tuteurs 5,7 et 8, et aux attaches 5′ et 7′ tout autre type d'organe de liaison susceptible de se déformer élastiquement, y compris des ensembles à cylindre et piston équipés d'un moyen de rappel.

L'invention apporte donc un corset formant siège d'un type simple et facile à réaliser, qui accroît sensiblement la liberté de mouvement du patient, ce qui est particulièrement important dans le cas d'un enfant handicapé physique.

## Revendications

1. Corset formant siège pour handicapé physique, ayant la forme générale d'une unique coquille ouverte en une matière rigide ou semi-rigide qui épouse au moins partiellement la forme du siège du patient, de son bassin, de son tronc, de ses bras, de ses jambes et/ou de sa tête et qui est apte à recevoir le patient en position assise, ce corset comprenant une partie formant siège (1,1'), une partie distincte (2,2') d'appui du bassin, une partie formant dossier (3,3') et deux parties de maintien latéral (4,4'), la partie formant dossier (3,3') étant solidaire par un organe de liaison (5,5') de la partie (2,2') d'appui du bassin, cette partie (2,2') étant elle-même solidaire par un organe de liaison (5,5') de la partie formant siège (1,1'), caractérisé en ce que les parties de maintien latéral (4,4') sont toutes deux solidaires par un organe de liaison (7,7') de la partie (2,2') d'appui du bassin, et en ce que tous les organes de liaison (5,5',7,7') sont déformables élastiquement sans la solicitation du patient.

2. Corset formant siège selon la revendication 1, caractérisé en ce que les organes de liaison (5,7) sont distincts des parties (1-4) du corset formant siège qu'ils réunissent et sont fixés sur celles-ci.

3. Corset formant siège selon la revendication 1, caractérisé en ce que au moins certains organes de liaison (5',7') font partie intégrante du corset et sont constitués par des parties de celui-ci dont la forme, les dimensions et/ou l'épaisseur sont telles qu'elles leur permettent de se déformer élastiquement.

## Claims

1. A corset forming a seat for a disabled person, taking the general form of a single open shell of a rigid or semi-rigid material which at least partially matches the seated shape of the patient, his pelvis, his torso, his arms, his legs and/or his head, and which is capable of accommodating the patient in the seated position, this corset comprising a seat-forming portion (1, 1'), a separate pelvis-supporting portion (2, 2'), a back-forming portion (3, 3') and two side support portions (4, 4'), the back-forming portion (3, 3') being firmly connected by a connecting member (5, 5') to the pelvis-supporting portion (2, 2'), the latter portion (2, 2') itself being firmly connected by a connecting member (5, 5') to the seat-forming portion (1, 1'), characterised in that the side support portions (4, 4') are both firmly connected by a connecting member (7, 7') to the pelvis-supporting portion (2, 2') and in that all the connecting members (5, 5', 7, 7') may be deformed elastically in response to prompting by the patient.

2. A seat-forming corset according to claim 1, characterised in that the connecting members (5, 7) are separate from the portions (1-4) of the seat-forming corset which they connect and are fixed thereto.

3. A seat-forming corset according to claim 1, characterised in that at least certain connecting members (5', 7') constitute an integral part of the corset and consist of parts thereof of which the shape, dimensions and/or thickness are such that said parts are capable of elastic deformation.

## Patentansprüche

1. Korsett in Form eines Sitzes für Körperbehinderte, welches die generelle Form einer einzelnen offenen Muschel aufweist, bestehend aus einem steifen oder halbsteifen Material, und welches zumindest zum Teil die Form des Gesäßes des Patienten annimmt, weiter die Form dessen Beckens, Rumpfs, Arme, Beine und/oder Kopfes, und welches für das Stützen des Patienten in Sitzposition geeignet ist, wobei das Korsett einen Gesäßteil (1, 1'), einen getrennten Teil zur Beckenstütze (2, 2'), einen die Rückenlehne bildenden Teil (3, 3') und zwei seitliche Stützelemente (4, 4') aufweist, und wobei weiter der die Rückenlehne bildende Teil (3, 3') mit Hilfe eines Verbindungselements (5, 5') fest mit dem Beckenstützteil (2, 2') verbunden ist und dieser Teil (2, 2') selbst mit Hilfe eines Verbindungselements (5, 5') mit dem Gesäßteil (1, 1')verbunden ist, dadurch gekennzeichnet, daß die seitlichen Stützelemente (4, 4') beide fest mit Hilfe eines Verbindungselements (7, 7') mit dem Teil zur Beckenstütze (2, 2') verbunden sind und alle Verbindungselemente (5, 5', 7, 7') ohne Krafteinwirkung durch den Patienten elastisch verformbar sind.

2. Korsett in Form eines Sitzes für Körperbehinderte nach Anspruch 1, dadurch gekennzeichnet, daß zumindest die Verbindungselemente (5, 7) getrennt von den den Sitz bildenden Teilen des Korsetts (1-4) sind, welche sich aneinander fügen und an diesen befestigt werden.

3. Korsett, welches einen Sitz für Körperbehinderte nach Anspruch 1 bildet, dadurch gekennzeichnet, daß zumindest bestimmte Verbindungselemente (5', 7') einen integralen Bestandteil des Korsetts bilden und aus Teilen des Korsetts bestehen, wobei die Form, Größe und Stärke der Korsetteile so beschaffen sind, daß sie eine elastische Verformung ermöglichen.
